(19) Europäisches Patentamt
European Patent Office
Office européen des brevets

(11) **EP 3 513 779 B1**

(12) **FASCICULE DE BREVET EUROPEEN**

(45) Date de publication et mention
de la délivrance du brevet:
**29.07.2020 Bulletin 2020/31**

(51) Int Cl.:
*A61K 8/02* (2006.01)          *A61K 8/37* (2006.01)
*A61K 8/85* (2006.01)          *A61Q 19/10* (2006.01)

(21) Numéro de dépôt: **18210991.8**

(22) Date de dépôt: **07.12.2018**

(54) **COMPOSITION COSMÉTIQUE COMPRENANT DES PARTICULES POLYMÈRES THERMOPLASTIQUES BIODÉGRADABLES, UTILISATION COSMÉTIQUE DE CES PARTICULES ET PROCÉDÉ DE TRAITEMENT COSMÉTIQUE**

KOSMETISCHE ZUSAMMENSETZUNG, DIE BIOLOGISCH ABBAUBARE THERMOPLASTISCHE POLYMERPARTIKEL ENTHÄLT, KOSMETISCHE VERWENDUNG DIESER PARTIKEL UND VERFAHREN ZUR KOSMETISCHEN BEHANDLUNG

COSMETIC COMPOSITION INCLUDING BIODEGRADABLE THERMOPLASTIC POLYMER PARTICLES, COSMETIC USE OF SAID PARTICLES AND COSMETIC TREATMENT METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.01.2018 FR 1870042**

(43) Date de publication de la demande:
**24.07.2019 Bulletin 2019/30**

(73) Titulaires:
• **Laboratoires Clarins**
  **75017 Paris (FR)**
• **Kairos**
  **29900 Concarneau (FR)**

(72) Inventeurs:
• **RAJAOMARIA, Rado**
  **92500 RUEIL-MALMAISON (FR)**
• **GROSSMANN, Erwan**
  **29350 MOELAN-SUR-MER (FR)**
• **KHALFALLAH, Moussa**
  **29900 Quimper (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**WO-A1-2017/027344      JP-A- 2016 222 897**
**US-A1- 2017 218 119**

• **Marlon Bustos: "Effect of Selected Additives on the Mechanical and Rheological Properties of an Amorphous Poly(Hydroxyalkanoate)", , 31 août 2017 (2017-08-31), pages FP-IX,1-59, XP002784876, Department of Chemical Engineering McGill University Montreal, Quebec, Canada Extrait de l'Internet: URL:http://digitool.library.mcgill.ca/webc lient/StreamGate?folder_id=0&dvs=153734697 7853~792 [extrait le 2018-09-19]**

EP 3 513 779 B1

**Description**

DOMAINE DE L'INVENTION

La présente invention concerne le domaine des matériaux polymères thermoplastiques biodégradables.

CONTEXTE DE L'INVENTION

Il y a actuellement une forte demande de la part des consommateurs mais également des industriels, notamment des industriels de la plasturgie, pour des matériaux polymères thermoplastiques toujours plus respectueux de l'environnement.

Il est connu de l'homme du métier que des pièces thermoplastiques moulées peuvent être fabriquées à partir de particules polymères thermoplastiques telles que des particules de polyéthylène, polypropylène ou polyamide. On peut notamment citer comme procédé standard de fabrication de telles pièces le procédé de moulage par rotation appelé également procédé de rotomoulage ou le procédé de moulage par injection appelé également procédé d'injection plastique.

Lors de la mise en œuvre du procédé de rotomoulage, les particules sont chargées dans un moule constitué de deux parties démontables. Une fois chargé et fermé, le moule est placé dans un four et tourne suivant deux axes orthogonaux pendant la phase de chauffage. Une fois la phase de chauffage terminée, le moule toujours en rotation est sorti du four afin d'être refroidi, l'eau, l'air froid ou une combinaison d'eau et d'air froid permettant le refroidissement du moule et donc de la matière fondue.

Lors de la mise en œuvre du procédé d'injection plastique, les particules sont chargées dans un doseur à trémie servant à alimenter une vis de plastification de type vis sans fin qui est contenue dans un fourreau chauffé et thermo régulé. La rotation de la vis de plastification (entraînée par un moteur hydraulique) et l'action conjuguée de la température du fourreau permettent de ramollir les particules et d'obtenir une matière fondue. La matière fondue est ensuite acheminée à l'avant de la vis de plastification, donnant ainsi une réserve de matière prête à être injectée, c'est la phase de dosage. Vient ensuite la phase d'injection dynamique, où la matière présente à l'avant de la vis de plastification est injectée sous forte pression à l'intérieur d'un moule présentant la forme de la pièce souhaitée, le moule étant régulé à une température inférieure à la température de transformation. Quand la matière plastique arrive au contact du moule, elle prend la forme du moule et se solidifie. Le moule est ensuite ouvert afin de faire sortir la pièce.

Dans ces deux types de procédé, les caractéristiques techniques des particules ont une influence directe sur la qualité de la pièce obtenue.

Les particules utilisées dans le procédé de rotomoulage sont traditionnellement obtenues par un procédé de micronisation permettant d'obtenir des particules de faible taille et de forme irrégulière. Or, il est préférable d'utiliser des particules sphériques ayant une granulométrie relativement homogène, idéalement entre 100 $\mu$m et 500 $\mu$m. Dans le cas du procédé de rotomoulage, de telles particules ont une vitesse de fusion plus homogène permettant une meilleure répartition de la matière fondue sur la paroi du moule et donc une épaisseur de la pièce moulée plus régulière.

De même, dans le cas du procédé de moulage par injection, l'utilisation de particules sphérique permet d'assurer une alimentation uniforme de la machine de moulage par injection permettant ainsi d'obtenir une densité et une qualité homogène des pièces moulées obtenues.

Un autre procédé de fabrication de particules polymères thermoplastiques est utilisé afin d'obtenir des particules sphériques ayant une granulométrie plus homogène. Il s'agit du procédé de granulation sous eau. Un tel procédé est décrit par exemple dans le brevet FR1247574. Ce procédé comprend l'extrusion d'un polymère ou d'une composition polymère thermoplastique, l'amenée de la matière extrudée, sous une pression appliquée continuellement, à une filière d'extrusion reliée à un organe coupant puis la coupe de la matière extrudée en particules distinctes de la matière extrudée. Les particules sont presque immédiatement mises en contact avec un courant d'un fluide sous pression qui ne réagit pas et n'est pas miscible avec la matière extrudée de sorte que les particules de matière plastique sont rapidement refroidies et solidifiées. Le fluide inerte utilisé dans ce procédé est typiquement un fluide aqueux tel que l'eau. Cependant, un des inconvénients majeur lié à ce type de particules réside dans leur faible biodégradabilité ce qui fait que les pièces obtenues à partir de ces particules par un procédé d'injection plastique ou par un procédé de rotomoulage sont également relativement peu biodégradables.

US 2017/0218119 décrit des particules de polyhydroxyalcanoate (PHA) produites par un procédé de granulation sous eau, éventuellement en présence d'un plastifiant. Par ailleurs, il est également connu de l'homme du métier que des particules polymères thermoplastiques telles que des particules de polyéthylène, polypropylène ou polyamide peuvent également être utilisées dans des produits cosmétiques afin de nettoyer la peau par élimination des impuretés et des cellules mortes. Ces particules sont incorporées principalement en suspension dans les formulations cosmétiques et dans les préparations similaires pour la toilette. Elles nettoient la peau par exfoliation mécanique, sous l'action de massage et de gommage. Elles permettent ainsi de lisser la peau et d'en améliorer l'éclat. Un des avantages de ces particules exfoliantes réside dans leur caractère non-toxique et non-irritant pour l'être humain.

Néanmoins, l'utilisation de ces particules en tant qu'agents exfoliants dans des formulations cosmétiques est de plus en plus controversée. En effet, en raison de leur faible taille, de l'ordre de 150 à 1500 μm et de leur faible biodégradabilité, elles peuvent se retrouver en fin de vie dans les océans, les rivières et les lacs et ainsi se révéler dangereuses pour l'écosystème et notamment la faune marine qui peut les ingérer.

Afin de contourner ce problème, des matériaux plus respectueux de l'environnement ont été utilisés pour fabriquer ces particules. C'est le cas par exemple des polymères d'hydroxyalcanoates, notamment des polymères d'hydroxybutyrate. On peut citer par exemple les agents exfoliants de la marque Bioscrub ® qui sont commercialisés par la société Micro Powders Inc. Ces particules sont fabriquées à partir de polyhydroxybutyrate (PHB) selon un procédé de micronisation. Ces particules sont certes caractérisées par un fort taux de biodégradabilité mais elles ont une forme irrégulière et sont creuses et concaves, ce qui ne facilite pas leur utilisation dans des procédés d'injection ou de rotomoulage tel qu'expliqué ci-avant. De plus, en raison de leur forme irrégulière, les particules peuvent être trop abrasives pour la peau ce qui n'est pas toujours souhaitable notamment si elles sont destinées à être incorporées dans des formulations cosmétiques exfoliantes pour lesquelles une exfoliation plus douce est recherchée.

Par conséquent, il existe toujours un besoin pour des particules polymères thermoplastiques qui sont caractérisées par un fort taux de biodégradabilité et qui peuvent être utilisées comme matière première dans des procédés d'injection plastique ou de rotomoulage pour la fabrication de pièces thermoplastiques moulées et également en tant qu'agents exfoliants dans des formulations cosmétiques.

De plus, il existe également un besoin pour un procédé d'obtention de ces particules qui est simple, peu couteux, plus respectueux de l'environnement et qui permet un meilleur contrôle de la forme et de la taille des particules obtenues, ce procédé permettant d'obtenir des particules sphériques et ayant une granulométrie homogène.

Les inventeurs ont trouvé de manière surprenante que l'ensemble de ces problèmes peuvent être résolus par les particules polymères thermoplastiques qui sont comprises dans la composition cosmétique selon la présente invention ou qui sont utilisées selon la présente invention comme agents d'exfoliation de la peau humaine.

RÉSUMÉ DE L'INVENTION

La présente invention a pour objet une composition cosmétique comprenant dans un milieu cosmétiquement acceptable des particules polymères thermoplastiques obtenues par un procédé comprenant les étapes suivantes :

a) préparation d'une composition comprenant :

- au moins un polymère thermoplastique choisi dans le groupe constitué des homopolymères d'hydroxyalcanoates, des copolymères d'hydroxyalcanoates et de leurs mélanges ; et
- au moins un plastifiant ; et

b) obtention de particules polymères thermoplastiques à partir de la composition préparée en a) par un procédé de granulation sous eau.

La présente invention à également pour objet l'utilisation cosmétique des particules définies obtenues par un procédé comprenant les étapes suivantes :

a) préparation d'une composition comprenant :

- au moins un polymère thermoplastique choisi dans le groupe constitué des homopolymères d'hydroxyalcanoates, des copolymères d'hydroxyalcanoates et de leurs mélanges ; et
- au moins un plastifiant ; et

b) obtention de particules polymères thermoplastiques à partir de la composition préparée en a) par un procédé de granulation sous eau ;

comme agents d'exfoliation de la peau humaine.

La présente invention concerne également un procédé de traitement des irrégularités visibles et/ou tactiles de la peau humaine comprenant les étapes suivantes :

i) application topique sur la peau d'une composition cosmétique telle que définie ci-dessus; et
ii) maintien de la composition au contact de la peau pendant une durée comprise entre 1 min et 30 min, de préférence entre 1 min et 5 min ; et

iii) élimination de la composition par rinçage.

BRÈVE DESCRIPTION DES FIGURES

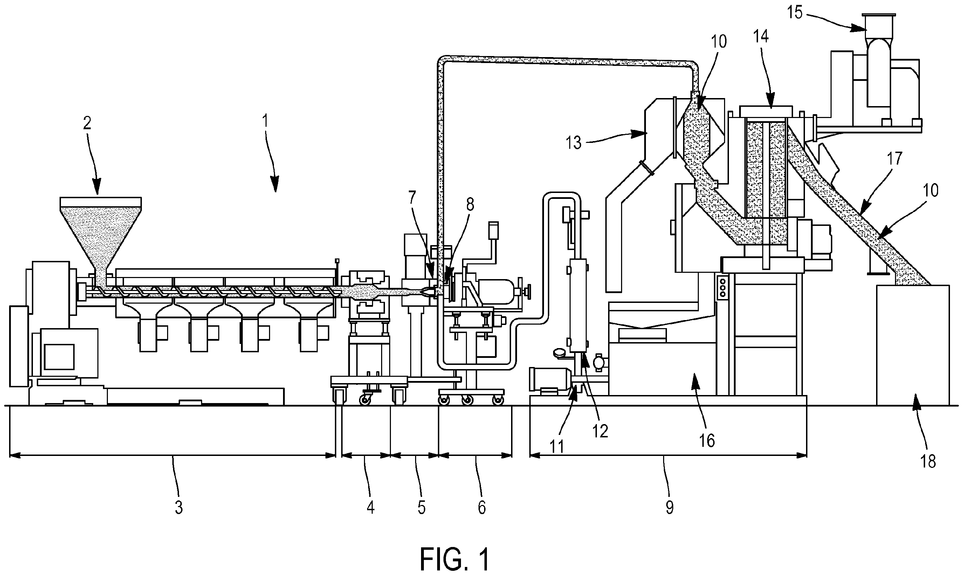

La Figure 1 représente un schéma d'ensemble d'une ligne de granulation sous eau permettant d'obtenir les particules polymères thermoplastiques comprises dans la composition cosmétique selon la présente invention ou utilisées comme agents d'exfoliation de la peau humaine selon la présente invention.

DESCRIPTION DETAILLÉE DE L'INVENTION

Par "charge", on entend toute substance inerte, d'origine végétale, minérale, organique ou synthétique qui est ajoutée à une composition polymère thermoplastique afin d'en modifier les propriétés mécaniques, électriques ou thermiques, ou afin d'améliorer l'aspect de surface ou bien de réduire le prix de revient du matériau obtenu à partir de la composition.

Par "agent de nucléation", on entend une particule insoluble qui est introduite dans une composition polymère thermoplastique afin d'en augmenter la vitesse de cristallisation.

Par "dérivés" d'un composé, on entend un analogue structurel de type ester, éther, amide, hydroxyle et/ou un sel du composé en question.

Par "circularité", on entend la forme d'une particule qui est mesurée sur la base d'une analyse de l'image en 2-dimension de cette particule selon la méthode ISO 9176-6:2008 (E), section 8.2. La circularité est souvent décrite dans la littérature comme la différence entre la forme de la particule et un cercle parfait. La circularité varie entre 0 et 1, une circularité de 1 décrivant des particules parfaitement sphérique ou dont la projection est parfaitement circulaire.

Par "diamètre de Féret" F, on entend la distance comprise entre une droite donnée D et la parallèle à cette direction de telle sorte que l'ensemble de la projection d'une particule soit comprise entre ces deux parallèles. En faisant tourner la droite D autour de la particule, le diamètre de Féret varie entre une valeur maximale appelée diamètre de Féret maximum Fmax et une valeur minimale appelée diamètre de Féret maximum Fmin. Le diamètre de Féret maximum Fmax et le diamètre de Féret maximum Fmin sont mesurés selon la méthode ISO 9176-6:2008 (E), section 8.1.2.

Par "rapport de forme", on entend le rapport Fmin/ Fmax, tel que défini dans la méthode ISO 9176-6:2008 (E), section 8.1.3.

Par "taille de particule", on entend le diamètre de cercle équivalent (ECD) de la particule tel que calculé selon la méthode ISO 9176-6:2008 (E), section 7.

Par milieu "cosmétiquement acceptable" on entend un milieu compatible avec les matières et/ou les fibres kératiniques d'êtres humains, comme par exemple, de manière non limitative, la peau, les muqueuses, les ongles, les cils, les sourcils, le cuir chevelu et/ou les cheveux.

Par "procédé de fabrication additive" on entend un procédé selon lequel des pièces thermoplastiques sont fabriquées par addition de couches successives de matière sous contrôle d'un ordinateur. On peut citer comme exemple de procédé de fabrication additive le procédé d'impression 3D.

Composition cosmétique comprenant des particules polymères thermoplastiques

La présente invention a pour objet une composition cosmétique comprenant dans un milieu cosmétiquement acceptable des particules polymères thermoplastiques obtenues par un procédé comprenant l'étape a) de préparation d'une composition comprenant au moins un polymère thermoplastique choisi dans le groupe constitué des homopolymères d'hydroxyalcanoates, des copolymères d'hydroxyalcanoates et de leurs mélanges et au moins un plastifiant et l'étape b) d'obtention de particules polymères thermoplastiques à partir de la composition préparée en a) par un procédé de granulation sous eau.

Les particules peuvent être présentes dans la composition cosmétique en un teneur totale en poids allant de 0,1 % à 10,0 %, de préférence de 0,3 % à 8,0 %, plus préférentiellement de 0,5 % à 5,0 %, encore plus préférentiellement de 1,0 % à 3,0 % par rapport au poids total de la composition.

La composition cosmétique peut comprendre au moins un solvant sélectionné dans le groupe constitué de l'eau, des alcools monohydriques, des alcools polyhydriques, de la glycérine, des glycols, des polyalkylène glycols et de leurs mélanges, de préférence dans le groupe constitué de l'éthanol, du propanol, du butanol, de l'isopropanol, du 1,2-propanediol, du 1,3-propanediol, du butanediol, du pentanediol, de l'hexanediol, de l'heptanediol, du decanediol, de la glycérine, de l'eau et de leurs mélanges.

La composition cosmétique peut comprendre au moins une huile. L'huile peut être sélectionnée dans le groupe constitué des silicones, des hydrocarbures, des esters, des amides gras, des éthers et de leurs mélanges.

La composition cosmétique peut comprendre au moins un agent structurant. Les agents structurants peuvent être utilisés pour suspendre ou disperser les particules abrasives. Les agents structurants sont généralement regroupés en fonction de leur solubilité, leur dispersibilité, ou la compatibilité de phase. Les agents structurants peuvent par exemple être choisis parmi les agents polymères, les gommes naturelles ou synthétiques et les polysaccharides.

La composition cosmétique peut avoir un pH variant entre 4,5 et 7, de préférence entre 4,7 et 6.

Utilisation cosmétique des particules polymères thermoplastiques

La présente invention a également pour objet l'utilisation cosmétique des particules polymères thermoplastiques obtenues par un procédé comprenant l'étape a) de préparation d'une composition comprenant au moins un polymère thermoplastique choisi dans le groupe constitué des homopolymères d'hydroxyalcanoates, des copolymères d'hydroxyalcanoates et de leurs mélanges et au moins un plastifiant et l'étape b) d'obtention de particules polymères thermoplastiques à partir de la composition préparée en a) par un procédé de granulation sous eau, comme agents d'exfoliation de la peau humaine.

Le polymère thermoplastique peut de préférence être choisi dans le groupe constitué du poly(3-hydroxybutyrate), du poly(3-hydroxyhexanoate), du poly(3-hydroxyvalérate), du poly(3-hydroxybutyrate-co-3-hydroxyvalérate), du poly(3-hydroxybutyrate-co-3-hydroxyhexanoate), du poly(3-hydroxybutyrate-co-3-hydroxyvalérate) et de leurs mélanges. Le polymère thermoplastique peut plus préférentiellement être le poly(3-hydroxybutyrate).

Les particules peuvent avoir une circularité allant de 0,90 à 1, de préférence de 0,98 à 1.

Les particules peuvent avoir un rapport de forme allant de 0,80 à 1, de préférence de 0,87 à 1.

Les particules peuvent avoir une taille allant de 250 $\mu$m à 1000 $\mu$m, de préférence de 250 $\mu$m à 500 $\mu$m.

Les particules obtenues par le procédé décrit ci-avant sont sphériques, de petite taille et ont une granulométrie relativement homogène, et conviennent donc particulièrement pour une utilisation comme agents exfoliants dans des compositions cosmétiques. Ces particules peuvent également être utilisées comme matière première dans des procédés d'injection plastique ou de rotomoulage.

Procédé de granulation sous eau

Comme expliqué ci-avant, le procédé de granulation sous eau, également appelé procédé de granulation en tête sous eau est un procédé connu de l'homme du métier. Un tel procédé est décrit par exemple dans le brevet FR1247574.

Étape a)

Au moins une portion, de préférence la totalité de la composition préparée en a) peut être préparée sous forme de granulés, les granulés étant préparés par un procédé de compactage ou de compoundage, de préférence par un procédé de compactage.

La composition préparée sous forme de granulés peut être une composition comprenant :

- au moins un polymère thermoplastique choisi dans le groupe constitué des homopolymères d'hydroxyalcanoates, des copolymères d'hydroxyalcanoates et de leurs mélanges ; et
- au moins un plastifiant.

Par "procédé de compactage", on entend un procédé d'agglomération d'un mélange homogène de poudres de polymères et de liquides par mise sous pression sur une filière. Ce procédé permet d'obtenir des granulés par réduction de volume de la poudre, tout en s'affranchissant d'une exposition de cette dernière à un cycle de température élevée pouvant entraîner sa dégradation. La présence d'un plastifiant sous forme liquide lors du compactage permet une meilleure cohésion de la poudre et donc une bonne tenue du granulé compacté.

Par "procédé de compoundage", on entend un procédé d'extrusion-granulation permettant le mélange par fusion d'un polymère avec un ou plusieurs additifs afin d'obtenir une matière plastique sous forme de compounds.

Les granulés sont préparés de préférence par un procédé de compactage. Un procédé de compactage est préférable au procédé de compoundage car lors du procédé de compoundage, le polymère est chauffé et cisaillé et risque donc de se dégrader.

Il est particulièrement avantageux d'utiliser une composition polymère sous forme de granulés. En effet, lorsque la composition est introduite sous d'autres formes telles que des poudres, il y a un risque que ces dernières se répandent dans l'atelier de production en raison d'une trop grande volatilité et donc puissent être inhalées par inadvertance lors de sa manipulation par un opérateur.

## Étape b)

L'étape b) du procédé peut comprendre la sous-étape $b_1$) d'obtention d'un mélange fondu par extrusion de la composition préparée en a) et la sous-étape $b_2$) de transfert du mélange fondu obtenu en $b_1$) vers un dispositif de coupe en tête sous eau 6 comprenant une filière 7 reliée à une tête de découpe sous eau 8, la filière 7 comprenant une pluralité d'orifices.

## $b_1$) Extrusion

L'extrusion peut être réalisée dans une extrudeuse 3. Une extrudeuse 3 comprend un fourreau cylindrique chauffant (thermorégulé) à l'intérieur duquel tourne une ou deux vis sans fin alimentée(s) en granulés ou en poudres de matières premières à l'aide de doseurs à trémie 2.
Par "extrudeuse monovis" on entend une extrudeuse comprenant une seule vis sans fin.
Par "extrudeuse bivis" on entend une extrudeuse comprenant deux vis sans fin.
La ou les vis sont constituées d'un ensemble d'éléments assemblés sur un arbre cannelé.
L'extrudeuse 3 utilisée en $b_1$) peut être choisie parmi les extrudeuses monovis, les extrudeuses bivis, les extrudeuses bivis coniques, les extrudeuses buss. L'extrudeuse 3 utilisée en $b_1$) peut être choisie de préférence parmi les extrudeuses bi-vis et les extrudeuses buss. En effet, il est préférable d'utiliser des extrudeuses bi-vis ou des extrudeuses buss notamment lorsqu'au moins une portion, de préférence la totalité de la composition préparée en a) est préparée sous forme de granulés car elles permettent d'avoir une extrusion réactive des composants et offrent une meilleure capacité de mélangeage et malaxage, en un temps de réaction rapide et en un temps de séjour court. Avantageusement, la technologie buss est plus adaptée aux polymères sensibles à la chaleur tels que les polyhydroxyalcanoates et permet une bonne maitrise des paramètres de mise en œuvre, notamment l'optimisation du couple temps-température.
Lors de l'extrusion, les différents éléments de la composition peuvent être conduits, à débit constant, à l'aide de doseurs à trémie 2 directement à l'intérieur de l'extrudeuse 3, où la chaleur et la pression peuvent être appliquées de manière à les faire fondre et obtenir un mélange fondu homogène. Le débit d'alimentation de l'extrudeuse 3 peut être d'au moins 3 kg par heures.
La (ou chacune des) vis de l'extrudeuse 3 est (sont) caractérisée(s) par son diamètre (D) et sa longueur (L) ainsi que par le ratio de ces deux paramètres (L/D). Le diamètre et le profil de la (ou de chacune des) vis d'extrusion peuvent être choisis en fonction des paramètres rhéologiques et physico chimiques de la composition polymère extrudée. Le rôle de la (ou de chacune des) vis consiste à mélanger, compresser, cisailler, échauffer et transporter en continu la composition fluidifié et homogénéisée.

## $b_2$) Transfert du mélange fondu

Le transfert du mélange fondu obtenu en $b_1$) vers le dispositif de coupe en tête sous eau 6 peut être facilité par l'insertion d'une pompe d'alimentation à engrenage 4 entre l'extrudeuse 3 et le dispositif de coupe en tête sous eau 6. La pompe d'alimentation 4 permet de faire avancer à débit constant le mélange obtenu en $b_1$) vers les orifices de la filière 7, dont le diamètre est sélectionné suivant la taille des particules désirées, et à le maintenir sous une pression continuelle dans la filière.
Le mélange fondu obtenu en $b_1$) peut être alimenté dans les orifices de la filière 7 sous une pression comprise entre 20 bars et 240 bars, de préférence entre 40 bars et 130 bars.

## Dispositif de coupe en tête sous eau 6

Le dispositif de coupe en tête sous eau 6 comprend une filière 7 reliée à une tête de découpe sous eau 8.

## Filière

La filière 7 comprenant une pluralité d'orifices.
Par "entrée de filière" on entend l'ensemble des extrémités des orifices de la filière 7 qui sont le plus proche de l'extrudeuse 3 lors de la mise en œuvre du procédé selon la présente invention.
Par "sortie de filière" on entend l'ensemble des extrémités des orifices de la filière 7 qui sont le plus proche de la tête de découpe sous eau 8 lors de la mise en œuvre du procédé selon la présente invention.
L'ensemble des orifices de la filière 7 peuvent de préférence avoir la même forme et les mêmes dimensions. L'ensemble des orifices de la filière 7 peuvent de préférence avoir une forme conique ou cylindrique, plus préférentiellement cylindrique.

Le diamètre maximum des orifices de la filière 7 peut être compris entre 0,25 mm et 1,0 mm. La longueur des orifices de la filière 7 peut être comprise entre 2,0 mm et 10,0 mm.

Il est particulièrement avantageux d'utiliser une filière 7 ayant des orifices de géométrie cylindrique car une telle filière est plus facile à fabriquer.

Dans le cas où l'ensemble des orifices de la filière ont une forme conique, la forme conique peut être une forme conique convergente ce qui signifie que le diamètre des orifices de la filière diminue entre l'entrée et la sortie de filière ou une forme conique divergente ce qui signifie que le diamètre des orifices de la filière augmente entre l'entrée et la sortie de filière.

Alternativement, les orifices de la filière peuvent avoir une forme qui change entre l'entrée et la sortie de filière. Dans ce cas-là entre l'entrée et la sortie de filière, les orifices de la filière peuvent avoir une forme qui change d'une forme conique convergent à une forme cylindrique ou d'une forme conique convergente à une forme cylindrique puis conique divergente ou d'une forme conique convergente à une forme cylindrique puis conique convergente.

Tête de découpe sous eau 8

La tête de découpe sous eau 8 peut être équipée d'un porte couteau comprenant une pluralité de lames, de préférence quatre lames. Lors de la mise en œuvre du procédé selon la présente invention, la tête de découpe sous eau peut avoir une vitesse de rotation d'au moins 500 tours/minute, de préférence au moins 3000 tours/minute.

Le fonctionnement de la ligne de microgranulation 1 est décrit ci-après dans la section exemples de la présente demande.

Les inventeurs ont remarqué de manière surprenante que l'utilisation d'un procédé de granulation sous eau permet d'obtenir des particules polymères thermoplastiques sphériques ayant une granulométrie relativement homogène à partir d'une composition comprenant au moins un polymère choisi dans le groupe constitué des homopolymères d'hydroxyalcanoates, des copolymères d'hydroxyalcanoates et de leurs mélanges. De telles particules peuvent ainsi être utilisées dans des procédés d'injection plastique ou de rotomoulage ou en tant qu'agents d'exfoliation de la peau humains dans des compositions cosmétiques tel qu'expliqué ci-avant.

Cependant, afin de pouvoir mettre en œuvre le procédé de granulation, il a été nécessaire de l'adapter à l'utilisation des polymères d'hydroxyalcanoates. En effet, lors des premiers essais, les inventeurs ont remarqué qu'il se produisait un phénomène de gonflement du polymère en sortie de filière. Ce phénomène de gonflement a pour conséquence qu'il est difficile de contrôler la taille des particules obtenues par le procédé de granulation sous eau et d'obtenir des particules ayant une granulométrie relativement homogène.

Ce phénomène de gonflement peut s'expliquer en raison de la nature même du polymère utilisé. En effet, lors du passage de la matière fondue du fourreau de l'extrudeuse (d'une section large) vers les orifices de la filière (d'une section étroite), le polymère subit une contrainte élongationnelle d'entrée qui, compte tenu de la viscoélasticité du matériau, ne peut se relaxer que faiblement lors de son séjour dans les orifices de la filière. Cette contrainte engendre, lors de la sortie de filière une recouvrance élastique plus ou moins complète entraînant un gonflement, correspondant à la suppression de l'état de déformation. On parle alors d'effet mémoire du polymère qui revient à son état initial d'avant son entrée dans la filière. Selon les inventeurs, ce phénomène du gonflement serait directement lié au comportement viscoélastique des polymères d'hydroxyalcanoates et plus particulièrement à la propriété purement élastique responsable du recouvrement de la déformation imposée à l'entrée de filière, c'est-à-dire le module d'élasticité. Les inventeurs ont trouvé de manière surprenante que l'ajout d'un plastifiant permet de réduire l'élasticité du polymère et donc de limiter son retour vers sa forme initiale en sortie de filière suite à la déformation imposée en entrée de filière et donc d'avoir un meilleur contrôle de la taille des particules obtenues en sortie de filière.

Composition préparée lors de l'étape a)

La composition préparée lors de l'étape a) du procédé comprend au moins un polymère thermoplastique choisi dans le groupe constitué des homopolymères d'hydroxyalcanoates, des copolymères d'hydroxyalcanoates et de leurs mélanges et au moins un plastifiant.

Polymère thermoplastique

Le polymère thermoplastique peut être présent dans la composition préparée en a) en une teneur totale en poids allant de 50% à 99%, de préférence de 60% à 85%, plus préférentiellement de 60% à 80%, le plus préférentiellement de 65% à 75% par rapport au poids total de la composition préparée en a).

Les particules peuvent avoir un taux de biodégradabilité d'au moins 90% au bout de 180 jours tel que mesuré selon la méthode de mesure de la biodégradabilité décrite ci-après.

Le polymère thermoplastique peut de préférence être choisi dans le groupe constitué du poly(3-hydroxybutyrate), du poly(3-hydroxyhexanoate), du poly(3-hydroxyvalérate), du poly(3-hydroxybutyrate-co-3-hydroxyvalérate), du poly(3-hydroxybutyrate-co-3-hydroxyhexanoate), du poly(3-hydroxybutyrate-co-3-hydroxyvalérate) et de leurs mélanges. Plus préférentiellement, le polymère thermoplastique peut être le poly(3-hydroxybutyrate).

Le polymère thermoplastique peut avoir une masse molaire allant de 300 000 g/mol à 400 000 g/mol.

Autres polymères

La composition préparée en a) peut comprendre en outre au moins un polymère sélectionné dans le groupe constitué des polymères biosourcés, biodégradables, compostables et de leurs mélanges.

Un même polymère peut être à la fois biosourcé et/ou biodégradable et/ou compostable.

Par "polymère biosourcé" on entend un polymère obtenu par extraction de la biomasse ou par des réactions appliquées à la biomasse.

Par "polymère biodégradable" on entend un polymère ayant un taux de biodégradabilité d'au moins 90% au bout de 180 jours tel que mesuré selon la méthode de mesure de la biodégradabilité décrite ci-après.

Par "polymère compostable" on entend un polymère pouvant être valorisé par compostage. Le compostage est un procédé qui consiste à placer des produits fermentescibles dans des conditions de températures, humidité, oxygénation, en présence de micro-organismes du sol permettant leur biodégradation. Les résidus de compostage ne doivent pas affecter la qualité finale du compost et le rendre, de ce fait, inapte pour une utilisation agricole.

La composition préparée en a) peut comprendre en outre au moins un polymère sélectionné dans le groupe constitué :

i) des homopolymères ou copolymères choisis dans le groupe constitué des dérivés d'acide polylactique, du polycaprolactone, de l'acide polyglycolique et de leurs mélanges;

ii) des co-polyesters aliphatiques et/ou aromatiques, les co-polyesters étant choisis de préférence parmi les co-polyesters contenant des monomères choisis dans le groupe constitué des monomères de l'acide succinique, de l'acide adipique, de l'acide téréphtalique, du propanediol, du butanediol, du pentanediol et des mélanges de ces monomères, plus préférentiellement parmi le succinate de polybutylène (PBS) et/ou le polybutylène-adipate-téréphtalate (PBAT);

iii) de l'amidon et/ou de l'amidon thermoplastique (TPS);

iv) des dérivés de cellulose thermoplastique (TPC) choisis dans le groupe constitué des esters de cellulose, des éthers de cellulose, des alcanoates et de leurs mélanges, de préférence dans le groupe constitué de l'acétate de cellulose et/ou de la nitrocellulose;

et des mélanges des composés i) à iv).

Plastifiant

Le plastifiant peut être présent dans la composition préparée en a) en une teneur totale en poids allant de 1% à 50 %, de préférence de 15% à 40%, plus préférentiellement de 20% à 40%, le plus préférentiellement de 25% à 35% par rapport au poids total de la composition préparée en a).

Le plastifiant peut être choisi dans le groupe constitué des esters d'acide citrique, des polyols, des polyoléfines, des huiles végétales, des surfactants et de leurs mélanges.

Les esters d'acide citrique peuvent de préférence être choisis dans le groupe constitué du citrate de triéthyle, du citrate de tributyle, de l'acétylcitrate de triéthyle, de l'acétylcitrate de tributyle et de leurs mélanges. Les polyols peuvent de préférence être choisis dans le groupe constitué du propylène glycol, du glycérol et de leurs mélanges. Les polyoléfines peuvent de préférence être choisies dans le groupe constitué du polyisobutylène, du phtalate de dibutyle, du sébacate de dibutyle et de leurs mélanges. Les huiles végétales peuvent de préférence être choisies parmi les triglycérides, plus préférentiellement dans le groupe constitué de l'huile de ricin, de l'huile de soja, de l'huile de soja epoxydée et de leurs mélanges. Les surfactants peuvent de préférence être choisis dans le groupe constitué des sophorolipides, du polyéthylène glycol et de leurs mélanges.

Le plastifiant peut être choisi dans le groupe constitué du citrate de triéthyle, du citrate de tributyle, de l'acétylcitrate de tributyle, de l'acétylcitrate de triéthyle et de leurs mélanges. Le plastifiant peut de préférence être le citrate de tributyle.

Chacun des plastifiants ou chacun des mélanges de plastifiants décrits ci-avant peut être compris dans la composition préparée en a) en combinaison avec chacun des homopolymères ou copolymères d'hydroxyalcanoates ou avec chacun des mélanges d'homopolymères et/ou de copolymères d'hydroxyalcanoates décrits ci-avant.

L'ajout d'un plastifiant permet non seulement d'avoir un meilleur contrôle de la taille des particules obtenues en sortie de filière tel qu'expliqué ci-dessus mais également de réduire la pression d'alimentation de la filière permettant l'écoulement de la matière fondue dans la filière ce qui permet de bonnes conditions de mise en œuvre du procédé selon la présente invention.

Charge(s)

La composition préparée en a) peut comprendre en outre au moins une charge choisie dans le groupe constitué des charges d'origine végétale, des charges d'origine minérale, des charges d'origine synthétique et de leurs mélanges, de préférence choisie dans le groupe constitué des charges d'origine végétale, des charges d'origine minérale et de leurs mélanges, plus préférentiellement parmi les charges d'origine végétale.

La charge peut être présente dans la composition préparée en a) en une teneur totale en poids allant de 1% à 50%, de préférence de 1% à 30%, plus préférentiellement de 5% à 15% par rapport au poids total de la composition préparée en a).

L'introduction de charges dans la composition selon la présente invention est particulièrement avantageuse car elle permet de réduire les coûts de revient des particules mais également d'améliorer les propriétés thermomécaniques et la stabilité dimensionnelle des pièces thermoplastiques moulées obtenues à partir de ces particules par un procédé d'injection plastique ou de rotomoulage en aidant à la création d'une armature permettant de renforcer la structure des pièces ainsi obtenues.

Les charges d'origine végétale peuvent être choisies dans le groupe constitué des composés ligno-cellulosiques, des farines de bois, des noyaux de fruits broyés, des écorces de fruits broyées et de leurs mélanges. Les charges d'origine végétale peuvent être choisies de préférence parmi les composés ligno-cellulosiques, plus préférentiellement parmi les celluloses, encore plus préférentiellement parmi les fibrilles de cellulose, le plus préférentiellement dans le groupe constitué des fibrilles de celluloses issues du lin, du chanvre, de l'avoine, du bois, du roseau, du miscanthus, du bambou ou de leurs mélanges.

Les charges d'origine minérale peuvent être choisies dans le groupe constitué des argiles, des micas, du talc, des craies, du carbonate de calcium, du carbonate de magnésium, des borosilicates, des perlites, des silices, du sulfate de baryum et de leurs mélanges.

Les argiles peuvent être des argiles naturelles ou des argiles modifiées. Les argiles peuvent être choisies dans le groupe constitué du kaolin, des halloysites et de leurs mélanges.

Les charges d'origine synthétique peuvent être choisies dans le groupe constitué, des microsphères de verre ou de silice synthétique, des nanotubes de carbones et de leurs mélanges.

Chacune des charges ou chacun des mélanges de charges décrits ci-avant peut être compris dans la composition préparée en a) en combinaison avec chacun des homopolymères ou copolymères d'hydroxyalcanoates ou avec chacun des mélanges d'homopolymères et/ou copolymères d'hydroxyalcanoates décrits ci-avant et en combinaison avec chacun des plastifiants ou chacun des mélanges de plastifiants décrits ci-avant.

À titre d'exemple, la composition préparée en a) selon la présente invention peut comprendre :

- au moins un polymère thermoplastique choisi dans le groupe constitué du poly(3-hydroxybutyrate), du poly(3-hydroxyhexanoate), du poly(3-hydroxyvalérate), du poly(3-hydroxybutyrate-co-3-hydroxyvalérate), du poly(3-hydroxybutyrate-co-3-hydroxyhexanoate), du poly(3-hydroxybutyrate-co-3-hydroxyvalérate) et de leurs mélanges ; et
- au moins un plastifiant choisi dans le groupe constitué du citrate de triéthyle, du citrate de tributyle, de l'acétylcitrate de tributyle, de l'acétylcitrate de triéthyle et de leurs mélanges, le plastifiant étant présent dans la composition préparée en a) en une teneur totale en poids allant de 1% à 50%, de préférence de 15% à 40%, plus préférentiellement de 20% à 40%, le plus préférentiellement de 25% à 35% par rapport au poids total de la composition préparée en a) ; et
- optionnellement au moins une charge choisie parmi les charges d'origine végétale, les charges d'origine végétale étant choisies dans le groupe constitué des composés ligno-cellulosiques, des farines de bois, des noyaux de fruits broyés, des écorces de fruits broyées et de leurs mélanges, de préférence parmi les composés ligno-cellulosiques, plus préférentiellement parmi les celluloses.

Alternativement, la composition préparée en a) selon la présente invention peut comprendre :

- au moins un polymère thermoplastique, le polymère thermoplastique étant le poly(3-hydroxybutyrate); et
- au moins un plastifiant choisi dans le groupe constitué du citrate de triéthyle, du citrate de tributyle, de l'acétylcitrate de tributyle, de l'acétylcitrate de triéthyle et de leurs mélanges, le plastifiant étant présent dans la composition préparée en a) en une teneur totale en poids allant de 1% à 50%, de préférence de 15% à 40%, plus préférentiellement de 20% à 40%, le plus préférentiellement de 25% à 35% par rapport au poids total de la composition préparée en a); et
- optionnellement au moins une charge choisie parmi les charges d'origine végétale, les charges d'origine végétale étant choisies dans le groupe constitué des composés ligno-cellulosiques, des farines de bois, des noyaux de fruits broyés, des écorces de fruits broyées et de leurs mélanges, de préférence parmi les composés ligno-cellulosiques, plus préférentiellement parmi les celluloses.

Alternativement, la composition préparée en a) selon la présente invention peut comprendre :

- au moins un polymère thermoplastique choisi dans le groupe constitué du poly(3-hydroxybutyrate), du poly(3-hydroxyhexanoate), du poly(3-hydroxyvalérate), du poly(3-hydroxybutyrate-co-3-hydroxyvalérate), du poly(3-hydroxybutyrate-co-3-hydroxyhexanoate), du poly(3-hydroxybutyrate-co-3-hydroxyvalérate) et de leurs mélanges ; et
- au moins un plastifiant, le plastifiant étant le citrate de tributyle, le plastifiant étant présent dans la composition préparée en a) en une teneur totale en poids allant de 1% à 50 %, de préférence de 15% à 40%, plus préférentiellement de 20% à 40%, le plus préférentiellement de 25% à 35% par rapport au poids total de la composition préparée en a) ; et
- optionnellement au moins une charge choisie parmi les charges d'origine végétale, les charges d'origine végétale étant choisies dans le groupe constitué des composés ligno-cellulosiques, des farines de bois, des noyaux de fruits broyés, des écorces de fruits broyées et de leurs mélanges, de préférence parmi les composés ligno-cellulosiques, plus préférentiellement parmi les celluloses.

Alternativement, la composition préparée en a) selon la présente invention peut comprendre :

- au moins un polymère thermoplastique, le polymère thermoplastique étant le poly(3-hydroxybutyrate); et
- au moins un plastifiant, le plastifiant étant le citrate de tributyle, le plastifiant étant présent dans la composition préparée en a) en une teneur totale en poids allant de 1% à 50%, de préférence de 15% à 40%, plus préférentiellement de 20% à 40%, le plus préférentiellement de 25% à 35% par rapport au poids total de la composition préparée en a) ; et
- optionnellement au moins une charge choisie parmi les charges d'origine végétale, les charges d'origine végétale étant choisies dans le groupe constitué des composés ligno-cellulosiques, des farines de bois, des noyaux de fruits broyés, des écorces de fruits broyées et de leurs mélanges, de préférence parmi les composés ligno-cellulosiques, plus préférentiellement parmi les celluloses.

Autres éléments

Agent de nucléation

La composition préparée en a) peut comprendre en outre au moins un agent de nucléation choisi dans le groupe constitué du nitrure de bore, de l'uracile, de la thymine, de la cytosine, de l'acide orotique, du chlorure d'ammonium, des cyclodextrines, des particules d'alcool polyvinylique, de l'oxyde de terbium, de la saccharine et de leurs mélanges. L'agent de nucléation peut de préférence être compris dans la composition préparée en a) en une teneur totale en poids allant de 0,1% à 15%, plus préférentiellement de 0,1% à 2% par rapport au poids total de la composition préparée en a).
Il est particulièrement avantageux d'ajouter un agent de nucléation dans la composition préparée en a) car l'agent de nucléation permet d'augmenter la vitesse de cristallisation des particules formées ce qui permet de limiter le risque de formation d'agrégats de particules en sortie de filière.

Pigment et/ou colorant

La composition préparée en a) peut comprendre en outre au moins un pigment et/ou au moins un colorant choisi(s) dans le groupe constitué du dioxyde de titane, de l'oxyde de zinc, des oxydes de fer, des sels ferrocyanures, des pigments organiques azoïques ou anthraquinones et de leurs mélanges, de préférence dans le groupe constitué du dioxyde de titane, de l'oxyde de zinc et de leur mélange.
Les oxydes de fer peuvent être de couleur jaune, rouge ou noire.
Le pigment et/ou le colorant peut/peuvent être présent(s) dans la composition préparée en a) en une teneur totale en poids allant de 0,5 % à 10 % par rapport au poids total de la composition préparée en a).
Il est particulièrement avantageux d'ajouter des pigments et/ou des colorants dans la composition préparée en a) car cela permet de colorer de manière uniforme les particules et de dissimuler les éventuellement imperfections de couleur par exemple dues à la présence de charges dans la composition.

Procédé de traitement cosmétique

La présente invention a également pour objet un procédé de traitement cosmétique des irrégularités visibles et/ou tactiles de la peau humaine comprenant les étapes suivantes :

i) application topique sur la peau d'une composition cosmétique selon la présente invention; et

ii) maintien de la composition au contact de la peau pendant une durée comprise entre 1 min et 30 min, de préférence entre 1 min et 5 min ; et
iii) élimination de la composition par rinçage.

Autres utilisations des particules polymères thermoplastiques

Les particules polymères thermoplastiques peuvent également être utilisées comme matière première pour la fabrication de pièces thermoplastiques moulées par un procédé d'injection plastique ou de rotomoulage. On peut citer comme exemples de pièces thermoplastiques moulées les pièces d'emballage, notamment les pièces d'emballage pour l'agroalimentaire.
Les particules polymères thermoplastiques peuvent également être utilisées comme matière première pour la fabrication de pièces thermoplastiques par un procédé de fabrication additive. Il est en effet particulièrement avantageux d'utiliser ces particules dans un procédé de fabrication additive tel que l'impression 3D car cela permet d'assurer un meilleur contrôle de l'écoulement de la matière lors de la mise en œuvre d'un tel procédé.

Méthode de mesure de la biodégradabilité

La biodégradabilité des particules polymères thermoplastiques peut être mesurée selon la méthode suivante. Il s'agit d'une méthode de mesure de la biodégradation aérobie des composés organiques dans une interface eau de mer/sédiments par analyse du dioxyde de carbone libéré selon la norme ISO/FDIS 19679.

1. Principe

La biodégradabilité des composés organiques par des micro-organismes en milieu marin est déterminée à l'aide d'un système d'essai statique. Dans un flacon de 250 mL, le mélange soumis à l'essai contient 80 mL d'eau de mer synthétique, 30 g de sédiment marin sec et 40 mg du composé organique à analyser. Le composé organique à analyser est placé en surface du sédiment marin, et maintenu avec de la toile nylon d'une porosité égale à 80 μm. Dans la suite de la description de cette méthode, cet échantillon est appelé "produit d'essai".

Eau de mer synthétique

L'eau de mer synthétique est une solution comprenant les ingrédients suivants dans un litre d'eau ultra-pure :

| Ingrédients | Quantités |
|---|---|
| Chlorure de sodium (NaCl) | 22 g |
| Chlorure de magnésium hexahydraté (MgCl$_2$, 6H$_2$O) | 9,7 g |
| Sulfate de sodium (Na$_2$SO$_4$) | 3,7 g |
| Chlorure de calcium anhydre (CaCl$_2$) | 1 g |
| Chlorure de potassium (KCl) | 0,65 g |
| Hydrogénocarbonate de sodium anhydre (NaHCO$_3$) | 0,20 g |

Le mélange est incubé à une température de 25 °C +/- 1 °C.
Le CO$_2$ formé pendant la dégradation microbienne est piégé dans des récipients contenant une solution d'hydroxyde de baryum et est déterminé par dosage titrimétrique deux à trois fois par semaine.
Le CO$_2$ libéré est comparé à la quantité de dioxyde de carbone théoriquement dégagée appelée également quantité de dioxyde de carbone théorique (CO$_2$Th) et exprimé en pourcentage. Le taux de biodégradation à un temps donné est calculé suivant l'équation suivante :

$$\text{Taux de biodégradation} = \frac{\text{CO}_2\text{cumulé (mg/xmL)}}{\text{CO}_2\text{Th (mg/xmL)}} \times 100$$

L'essai peut durer jusqu'à 6 mois mais peut être arrêté lorsque la phase de plateau est atteinte.

11

CO2Th : Quantité de dioxyde de carbone théorique

La quantité de dioxyde de carbone théorique est la quantité de dioxyde de carbone, calculée à partir de la teneur en carbone organique connue ou mesurée du composé organique à analyser, qui doit se dégager lors de la minéralisation complète de celui-ci ; le $CO_2Th$ en mg est calculé suivant l'équation suivante:

$$CO_2Th = \frac{44}{12} \times V_L \times \rho_c$$

dans laquelle :

44 et 12 sont respectivement la masse moléculaire relative du $CO_2$ et la masse atomique du carbone, destinées à calculer la quantité de $CO_2$ à partir du carbone organique du composé organique à analyser;
$V_L$ est le volume de solution d'essai contenu dans le flacon d'essai exprimé en litres;
pc est la concentration en carbone organique du composé organique à analyser dans le récipient d'essai en mg/L.
La teneur en carbone organique du composé organique à analyser est mesurée selon la norme NF EN 15407.

2. Mesures

Trois différents types d'échantillons sont préparés :

• Trois répliques du produit d'essai tel que défini ci-avant ;
• Trois répliques d'un témoin positif préparé de la même manière que le produit d'essai, le composé organique à analyser étant remplacé par une substance de référence : un filtre Whatman n°42 ;
• Trois répliques d'un témoin négatif préparé de la même manière que le produit d'essai, le composé organique à analyser n'étant pas présent.

3. Critères de validité du test

Le test est considéré comme validé si le pourcentage de biodégradation de la substance de référence a atteint au moins 60 % avant 180 jours et le dégagement total de $CO_2$ moyen du témoin négatif n'excède pas 3,5 mg $CO_2$/g de sédiment sec milieu (soit 105 mg pour 30 g) à la fin de l'essai.

EXEMPLES

L'invention est illustrée plus en détail par les exemples non limitatifs présentés ci-après. En l'absence d'indication contraire, les pourcentages indiqués sont des pourcentages massiques.

Ligne de procédé de granulation sous eau

La ligne de procédé de granulation sous eau 1 utilisée pour la réalisation du procédé de fabrication des particules polymères thermoplastiques est représentée en Figure 1. Elle se compose des éléments suivants :

- un dispositif d'alimentation en composition polymère ou doseur à trémie 2 ;
- une extrudeuse 3 ;
- une pompe d'alimentation à engrenage avec pression et débit ajustable (avec filtre) 4;
- une vanne d'orientation de la matière fondue vers la filière 5
- un dispositif de coupe en tête sous eau 6 comprenant :

    ▪ une filière comprenant une pluralité d'orifices 7,
    ▪ une tête de découpe sous eau 8,
    ▪ un système de circuit d'eau tempérée 9 circulant avec un débit constant et ajustable, permettant de conduire les particules 10 découpées vers un dispositif de séchage et

- un dispositif de séchage 14.

Le système de circuit d'eau tempérée 9 comprend une pompe 11, un échangeur de chaleur 12 ainsi qu'un dispositif de rétention d'aggloméré 13. Le dispositif de séchage 14 comprend un dispositif de ventilation 15.

La pompe d'alimentation à engrenage 4 est un dispositif d'alimentation optionnel dans la ligne de procédé de granulation sous eau 1, et n'est pas nécessaire si la rhéologie de la composition polymère utilisée permet un écoulement facile et à basse pression à travers l'extrudeuse 3 et la filière 7.

L'eau du système de circuit d'eau tempérée 9 circule à débit constant et à une température comprise entre 5°C et 100°C.

Le dispositif de séchage 14 est un dispositif de séchage par centrifugation.

Fonctionnement de la ligne de procédé de granulation sous eau 1

Au démarrage de la ligne de procédé de granulation sous eau 1 et pendant la mise en chauffe de ses différents éléments précités, le mélange fondu obtenu en $b_1$) sortant de la pompe d'alimentation à engrenage 4 est dirigé par une vanne d'orientation 5 vers un récipient de récupération. Après stabilisation des températures, la vanne 5 est actionnée de manière à orienter le mélange fondu obtenu en $b_1$) dans les orifices de la filière 7. La filière 7 est chauffée de préférence à une température variant entre 155°C et 200°C, correspondant à la température de fusion du polymère contenu dans la composition. La température de l'eau de refroidissement est réglée de préférence de 40°C à 80°C. La tête de découpe sous eau 7 est mise en marche simultanément, la vitesse de coupe (vitesse de rotation des lames) étant réglée de préférence à 5000 tours/minute afin d'obtenir le diamètre des particules désiré.

Le mélange fondu obtenu en $b_1$) est alimenté dans les orifices de la filière 7 sous une pression comprise entre 2000000 Pa et 24000000 Pa, de préférence entre 4000000 Pa et 13000000 Pa. Au démarrage du procédé de granulation, la pression d'alimentation du mélange fondu obtenu en $b_1$) dans les orifices de la filière 7 est établie à au moins 50% de sa valeur nominale en un temps très court avant de se stabiliser sensiblement au cours du procédé entre 4000000 Pa et 13000000 Pa.

Après découpe, les particules 10 sont conduites par le circuit d'eau tempérée 9 vers le dispositif de séchage par centrifugation 14. Dans un dispositif de séchage par centrifugation, l'eau est séparée des particules 10 et reste dans le circuit d'eau tempérée 9 et s'accumule dans un réservoir 16, sa température étant maintenue constante par un échangeur de chaleur 12. Les particules séchées 10 sont ensuite transférées à travers un canal 17 vers des bacs de récupération 18.

Fabrication de particules polymères thermoplastiques

Des particules polymères thermoplastiques ont été préparées à partir des compositions 1 et 2 suivantes en suivant le mode opératoire décrit ci-après:

| | poly(3-hydroxybutyrate)[1] | Citrate de tributyle[2] | Cellulose[3] | Nitrure de bore[4] | Dioxyde de titane[5] |
|---|---|---|---|---|---|
| **Composition 1** | 77,5 % | 10 % | 5 % | 0,5% | 7 % |
| **Composition 2** | 82,5 % | 10 % | 0 | 0,5% | 7 % |
| [1]Biomer ® biopolyester commercialisé par la société Biomer<br>[2]Citrofol ® B1 commercialisé par la société Jungbunzlauer<br>[3]Vitacel HF 101 commercialisé par la société JRS<br>[4]Nitrure de bore commercialisé par la société Sigma Aldrich<br>[5]Dioxyde de titane e171 commercialisé par la société Colorey SAS | | | | | |

Mode opératoire

Le mélange poly(3-hydroxybutyrate), citrate de tributyle, cellulose (si présent) et nitrure de bore est préparé sous forme de granulés par compactage.

Les granulés compactés mélangés avec le dioxyde de titane sont extrudés dans une extrudeuse bivis Leistritz ® (longueur 36D (36 * 50 mm), vis « soft »). La température de l'extrudeuse est de 175°C $\pm$ 5°C et la pression dans l'extrudeuse est de 160 bars $\pm$ 10 bars. Le temps de séjour dans l'extrudeuse est d'environ 4 minutes. Lors de cette étape, l'ensemble des composants (granulés compactés + dioxyde de titane) sont mélangés, compressés, cisaillés, échauffés afin d'obtenir un mélange fondu qui est transféré en continu et de manière homogène vers la pompe d'alimentation à engrenage grâce au mouvement rotationnel des vis d'extrusion.

Le mélange fondu sortant de la pompe d'alimentation à engrenage est ensuite dirigé par la vanne d'orientation vers les orifices de la filière du dispositif de coupe en tête sous eau. Le dispositif de coupe en tête sous eau utilisé est le modèle 6 A5 de marque Gala ®. Les orifices de la filière sont cylindriques et tous de même forme. La filière comprend 336 orifices ayant chacun un diamètre de 0,36 mm et une longueur de 4 mm ± 0,5 mm. Lors de la mise en œuvre du procédé, seuls 50% des orifices sont ouverts. La filière est chauffée à une température de 190°C ± 5°C. Le mélange fondu est alimenté dans les orifices de la filière sous une pression d'environ 11000000 Pa. Au démarrage du procédé de granulation, la pression d'alimentation du mélange fondu dans les orifices de la filière est établie à au moins 50% de sa valeur nominale en un temps très court avant de se stabiliser sensiblement au cours du procédé entre 1000000 Pa et 12000000 Pa. La tête de découpe sous eau comprenant 12 lames est mise en marche simultanément, la vitesse de coupe (vitesse de rotation des lames) étant réglée à 5000 tours/minute pour obtenir le diamètre des particules désiré de 400 μm ± 50 μm. La température de l'eau de refroidissement est réglée à 65°C.

Après découpe, les particules sont conduites par le circuit d'eau tempérée vers le dispositif de séchage par centrifugation. Les particules séchées sont ensuite transférées à travers le canal vers les bacs de récupération.

Les particules obtenues à partir de la composition 2 ont une circularité de 0,99 ± 0,01 et un rapport de forme de 0,87 ± 0,07.

Compositions cosmétiques comprenant des particules polymères thermoplastiques

Une crème exfoliante et un gel moussant exfoliant comprenant des particules polymères thermoplastiques ont été préparées à partir des compositions 1 et 2 selon le procédé décrit ci-avant.

Crème exfoliante

| Ingrédient | Quantité (%) |
|---|---|
| Stéarate de glycérol | 8,90 |
| Caprylic/capric triglycéride | 5,21 |
| Dimethicone | 5,21 |
| Butylene glycol | 5,21 |
| Sodium coco-sulfate | 4,48 |
| Alcool cétylique | 2,00 |
| Polyglyceryl-3-diisostearate | 1,77 |
| Cocamide Mipa | 0,90 |
| Acide citrique | 0,37 |
| Agent de texture | 0,47 |
| Conservateurs | 0,65 |
| EDTA disodique | 0,10 |
| Particules polymères thermoplastiques obtenues à partir de la composition 1 ou 2 | 10,00 |
| Eau | Q.s.p. 100 |

Gel moussant exfoliant

| Ingrédient | Quantité (%) |
|---|---|
| Sodium Laureth sulfate | 16,00 |
| Acrylates Copolymer | 5,20 |

(suite)

| Ingrédient | Quantité (%) |
|---|---|
| Cocamidopropyl Betaine | 5,00 |
| Decyl Glucoside | 2,50 |
| Extrait végétal | 1,00 |
| Acide citrique | 0,28 |
| EDTA disodique | 0,10 |
| Guar hydroxypropyltrimonium chloride | 0,05 |
| Hydroxyde de sodium | 0,05 |
| Conservateurs | 0,9 |
| Particules polymères thermoplastiques obtenues à partir de la composition 1 ou 2 | 10,00 |
| Eau | Q.s.p. 100 |

Mesure du module d'élasticité

Le module d'élasticité des compositions 3 et 4 suivantes a été mesuré à l'aide d'un rhéomètre rotationnel plan-plan RDA2 de marque Texas Instrument ®. Ces compositions peuvent aussi être utilisées afin de fabriquer des particules polymères thermoplastiques dans un procédé selon la présente invention.

| | poly(3-hydroxybutyrate)[1] | Citrate de tributyle[2] | Cellulose[3] | Nitrure de bore[4] | Dioxyde de titane[5] |
|---|---|---|---|---|---|
| Composition 3 | 72,5 % | 15 % | 5 % | 0,5% | 7% |
| Composition 4 | 62,5 % | 25 % | 5 % | 0,5% | 7% |
| [1]Biomer ® biopolyester commercialisé par la société Biomer<br>[2]Citrofol ® B1 commercialisé par la société Jungbunzlauer<br>[3]Vitacel HF 101 commercialisé par la société JRS<br>[4]Nitrure de bore commercialisé par la société Sigma Aldrich<br>[5]Dioxyde de titane e171 commercialisé par la société Colorey SAS | | | | | |

• Résultats

| | Module d'élasticité (Pa) mesuré à t = 20s |
|---|---|
| Composition 3 | 156 |
| Composition 4 | 74 |

• Conclusion

L'ajout d'un plastifiant à la composition à partir de laquelle les particules polymères thermoplastiques sont préparées permet de réduire le module d'élasticité de la composition.

Mesure du gonflement diamétral en sortie de filière en fonction de la pression d'alimentation de la filière

Le gonflement diamétral des compositions 5, 6 et 7 décrites ci-après a été mesuré à l'aide d'un rhéomètre capillaire afin de déterminer l'influence de la présence de plastifiant dans la composition sur le gonflement diamétral du polymère en sortie de filière.

|  | poly(3-hydroxybutyrate)[1] | Citrate de tributyle[2] | Cellulose[3] | Nitrure de bore[4] | Dioxyde de titane[5] |
|---|---|---|---|---|---|
| Composition 5 | 82,5 % | 10 % | 0 | 0,5% | 7% |
| Composition 6 | 72,5 % | 20 % | 0 | 0,5% | 7% |
| Composition 7 | 62,5 % | 30 % | 0 | 0,5% | 7% |
| [1]Biomer ® biopolyester commercialisé par la société Biomer<br>[2]Citrofol ® B1 commercialisé par la société Jungbunzlauer<br>[3]Vitacel HF 101 commercialisé par la société JRS<br>[4]Nitrure de bore commercialisé par la société Sigma Aldrich<br>[5]Dioxyde de titane e171 commercialisé par la société Colorey SAS | | | | | |

Ces compositions peuvent aussi être utilisées afin de fabriquer des particules polymères thermoplastiques dans un procédé selon la présente invention.

• Mode opératoire

Le rhéomètre capillaire utilisé est un rhéomètre capillaire bi-canal de type RH7 de la société Malvern.
Les mélanges fondus des compositions 5, 6 et 7 sont poussés par l'intermédiaire d'un piston, à vitesse contrôlée croissante à travers une filière à un orifice ayant 0,5 mm de diamètre et 8 mm de longueur. La filière est maintenue à une température de 175°C. Le gonflement diamétral est défini comme étant le rapport du diamètre du jonc en sortie de filière par rapport au diamètre de la filière. La variation du gonflement diamétral en fonction de la pression appliquée est mesurée successivement sur deux échantillons distincts de chacune des compositions pour une gamme de taux de cisaillement allant de 50 s$^{-1}$ à 10$^4$ s$^{-1}$ afin d'appréhender le comportement à l'état fondu de chacune des compositions.
La moyenne, l'écart-type ($\sigma$), la valeur minimale (G min), la valeur maximale (G max) du gonflement diamétral ainsi que la valeur minimale (P min) et la valeur maximale (P max) de la pression d'alimentation de la filière sont calculés pour chacune des compositions. Le coefficient de détermination linéaire ($R^2$) est également calculé pour chacune des compositions.

• Résultats

|  | Moyenne | $\sigma$ | G min | G max | P min (bars) | P max (bars) | $R^2$ |
|---|---|---|---|---|---|---|---|
| Composition 5 | 1,26 | 0,06 | 1,04 | 1,42 | 113,00 | 283,73 | 0,46 |
| Composition 6 | 1,34 | 0,04 | 1,20 | 1,44 | 43,78 | 184,31 | 0,30 |
| Composition 7 | 1,41 | 0,16 | 1,08 | 1,70 | 32,66 | 81,12 | 0,92 |

• Conclusion

L'ajout d'un plastifiant à la composition à partir de laquelle les particules polymères thermoplastiques sont préparées permet de réduire la pression d'alimentation de la filière ce qui permet une mise en œuvre du procédé dans de bonnes conditions en évitant les variations de pression trop importantes et l'augmentation de la pression à un seuil critique qui peut entrainer l'arrêt complet de la ligne de procédé de granulation et par conséquent le blocage de la filière en raison de la dégradation de la composition dans les orifices de la filière. L'ajout d'un plastifiant permet également un meilleur contrôle de la taille des particules obtenues en sortie de filière. En effet, en choisissant la bonne pression d'alimentation de la filière, il est possible de limiter le phénomène de gonflement et d'avoir un meilleur contrôle de la taille des particules obtenues en sortie de filière.

Mesure de la biodégradabilité

La biodégradabilité des particules polymères thermoplastiques préparées à partir de la composition 2 a été mesurée selon la méthode de mesure de la biodégradabilité décrite ci-avant.
Le sédiment marin qui a été utilisé pour effectuer la mesure est un sédiment marin prélevé à Gravelines (France) le 5 juin 2016. La teneur en carbone organique du sédiment est de 1,7%.
Les valeurs moyennes des dégagements théoriques en $CO_2$ ($CO_2Th$) mesurées sur 40 mg d'échantillon dans 100 mL de mélange (sédiment sec + eau de mer synthétique) sont égales à :

- 76,1 mg pour le produit d'essai ; et
- 62,0 mg pour le témoin positif.

Les particules ont un pourcentage de biodégradation de 90% à 81 jours.

## Revendications

1. Composition cosmétique comprenant dans un milieu cosmétiquement acceptable des particules polymères thermoplastiques obtenues par un procédé comprenant les étapes suivantes :

   a) préparation d'une composition comprenant :

      - au moins un polymère thermoplastique choisi dans le groupe constitué des homopolymères d'hydroxyalcanoates, des copolymères d'hydroxyalcanoates et de leurs mélanges ; et
      - au moins un plastifiant ; et

   b) obtention de particules polymères thermoplastiques à partir de la composition préparée en a) par un procédé de granulation sous eau.

2. Composition selon la revendication 1 dans laquelle :

   - le polymère thermoplastique est présent dans la composition préparée en a) en une teneur totale en poids allant de 50% à 99%, de préférence de 60% à 85%, plus préférentiellement de 60% à 80%, le plus préférentiellement de 65% à 75% par rapport au poids total de la composition préparée en a); et/ou
   - le plastifiant est présent dans la composition préparée en a) en une teneur totale en poids allant de 1% à 50 %, de préférence de 15% à 40%, plus préférentiellement de 20% à 40%, le plus préférentiellement de 25% à 35% par rapport au poids total de la composition préparée en a).

3. Composition selon l'une quelconque des revendications précédentes dans laquelle la composition préparée en a) comprend en outre au moins une charge choisie dans le groupe constitué des charges d'origine végétale, des charges d'origine minérale, des charges d'origine synthétique et de leurs mélanges, de préférence dans le groupe constitué des charges d'origine végétale, des charges d'origine minérale et de leurs mélanges, plus préférentiellement parmi les charges d'origine végétale.

4. Composition selon la revendication 3 dans laquelle:

   - les charges d'origine végétale sont choisies dans le groupe constitué des composés ligno-cellulosiques, des farines de bois, des noyaux de fruits broyés, des écorces de fruits broyées et de leurs mélanges, de préférence parmi les composés ligno-cellulosiques, plus préférentiellement parmi les celluloses, encore plus préférentiellement parmi les fibrilles de cellulose, le plus préférentiellement parmi les fibrilles de celluloses issues du lin, du chanvre, de l'avoine, du bois, du roseau, du miscanthus, du bambou ou de leurs mélanges; et/ou
   - les charges d'origine minérale sont choisies dans le groupe constitué des argiles, des micas, du talc, des craies, du carbonate de calcium, du carbonate de magnésium, des borosilicates, des perlites, des silices, du sulfate de baryum et de leurs mélanges.

5. Composition selon la revendication 3 ou 4, dans laquelle la charge est présente dans la composition préparée en a) de préférence en une teneur totale en poids allant de 1% à 50%, de préférence de 1% à 30%, plus préférentiellement de 5% à 15% par rapport au poids total de la composition préparée en a).

6. Composition selon l'une quelconque des revendications précédentes, dans laquelle le plastifiant est choisi dans le groupe constitué des esters d'acide citrique, des polyols, des polyoléfines, des huiles végétales, des surfactants et de leurs mélanges.

7. Composition selon l'une quelconque des revendications précédentes, dans lequel le plastifiant est choisi dans le groupe constitué du citrate de triéthyle, du citrate de tributyle, de l'acétylcitrate de tributyle, de l'acétylcitrate de triéthyle et de leurs mélanges, de préférence le citrate de tributyle.

8. Composition selon l'une quelconque des revendications précédentes, dans laquelle le polymère thermoplastique est choisi dans le groupe constitué du poly(3-hydroxybutyrate), du poly(3-hydroxyhexanoate), du poly(3-hydroxyvalérate), du poly(3-hydroxybutyrate-co-3-hydroxyvalérate), du poly(3-hydroxybutyrate-co-3-hydroxyhexanoate), du poly(3-hydroxybutyrate-co-3-hydroxyvalérate) et de leurs mélanges, de préférence le poly(3-hydroxybutyrate).

9. Composition selon l'une quelconque des revendications précédentes, dans laquelle le polymère thermoplastique a une masse molaire allant de 300 000 g/mol à 400 000 g/mol.

10. Composition selon l'une quelconque des revendications précédentes, dans laquelle la composition préparée en a) comprend en outre au moins un polymère sélectionné dans le groupe constitué :

    i) des homopolymères ou copolymères choisis dans le groupe constitué des dérivés d'acide polylactique, du polycaprolactone, de l'acide polyglycolique et de leurs mélanges;
    ii) des co-polyesters aliphatiques et/ou aromatiques;
    iii) de l'amidon et/ou de l'amidon thermoplastique (TPS);
    iv) des dérivés de cellulose thermoplastique (TPC) choisis dans le groupe constitué des esters de cellulose, des éthers de cellulose, des alcanoates et de leurs mélanges;

    et des mélanges des composés i) à iv).

11. Composition selon l'une quelconque des revendications précédentes dans laquelle la composition obtenue en a) comprend en outre au moins un agent de nucléation choisi dans le groupe constitué du nitrure de bore, de l'uracile, de la thymine, de la cytosine, de l'acide orotique, du chlorure d'ammonium, des cyclodextrines, des particules d'alcool polyvinylique, de l'oxyde de terbium, de la saccharine et de leurs mélanges.

12. Composition selon l'une quelconque des revendications précédentes dans laquelle la composition obtenue en a) comprend en outre au moins un pigment et/ou au moins un colorant choisi(s) dans le groupe constitué du dioxyde de titane, de l'oxyde de zinc, des oxydes de fer, des sels ferrocyanures, des pigments organiques azoïques ou anthraquinones et de leurs mélanges, de préférence dans le groupe constitué du dioxyde de titane, de l'oxyde de zinc et de leur mélange.

13. Composition selon l'une quelconque des revendications précédentes, dans laquelle au moins une portion, de préférence la totalité de la composition préparée en a) est préparée sous forme de granulés, les granulés étant préparés par un procédé de compactage ou de compoundage, de préférence par un procédé de compactage.

14. Composition selon l'une quelconque des revendications précédentes, dans laquelle l'étape b) du procédé comprend les sous-étapes suivantes :

    $b_1$) obtention d'un mélange fondu par extrusion de la composition préparée en a) ; et
    $b_2$) transfert du mélange fondu obtenu en $b_1$) vers un dispositif de coupe en tête sous eau (6) comprenant une filière (7) reliée à une tête de découpe sous eau (8), la filière (7) comprenant une pluralité d'orifices.

15. Composition selon la revendication précédente, dans laquelle lors de l'étape $b_2$), le mélange fondu obtenu en $b_1$) est alimenté dans les orifices de la filière (7) sous une pression comprise entre 2000000 Pa et 24000000 Pa, de préférence entre 4000000 Pa et 13000000 Pa.

16. Composition selon la revendication 14 ou 15, dans laquelle l'ensemble des orifices de la filière (7) ont la même forme et les mêmes dimensions, la forme étant de préférence conique ou cylindrique, plus préférentiellement cylindrique.

**17.** Composition selon l'une quelconque des revendications 14 à 16, dans laquelle :

- le diamètre maximum des orifices de la filière (7) est compris entre 0,25 mm et 1,0 mm; et
- la longueur des orifices de la filière (7) est comprise entre 2,0 mm et 10,0 mm.

**18.** Composition selon l'une quelconque des revendications précédentes, dans laquelle les particules ont:

- une circularité allant de 0,90 à 1, de préférence de 0,98 à 1 ; et
- un rapport de forme allant de 0,80 à 1, de préférence de 0,87 à 1 ; et
- une taille allant de 250 $\mu$m à 1000 $\mu$m, de préférence de 250 $\mu$m à 500 $\mu$m.

**19.** Utilisation cosmétique des particules polymères thermoplastiques telles que définies dans l'une quelconque des revendications précédentes comme agents d'exfoliation de la peau humaine.

**20.** Procédé de traitement cosmétique des irrégularités visibles et/ou tactiles de la peau humaine comprenant les étapes suivantes :

i) application topique sur la peau d'une composition cosmétique telle que définie dans l'une quelconque des revendications 1 à 18; et

ii) maintien de la composition au contact de la peau pendant une durée comprise entre 1 min et 30 min, de préférence entre 1 min et 5 min ; et

iii) élimination de la composition par rinçage.


## Patentansprüche

**1.** Kosmetikzusammensetzung, die in einem kosmetisch annehmbaren Medium thermoplastische Polymerpartikel umfasst, erhalten durch ein Verfahren, das die folgenden Schritte umfasst:

a) Herstellung einer Zusammensetzung, umfassend:

- mindestens ein thermoplastisches Polymer, ausgewählt aus der Gruppe bestehend aus Homopolymeren von Hydroxyalkanoaten, Copolymeren von Hydroxyalkanoaten und deren Gemischen; und
- mindestens einen Weichmacher; und

b) Erhalten thermoplastischer Polymerpartikel aus der unter a) hergestellten Zusammensetzung durch ein Unterwassergranulationsverfahren.

**2.** Zusammensetzung nach Anspruch 1, wobei:

- das thermoplastische Polymer in der unter a) hergestellten Zusammensetzung in einem Gesamtgehalt bezogen auf Gewicht von 50% bis 99%, vorzugsweise von 60% bis 85%, stärker bevorzugt von 60% bis 80%, am stärksten bevorzugt von 65% bis 75%, bezogen auf das Gesamtgewicht der unter a) hergestellten Zusammensetzung, vorliegt und/oder
- der Weichmacher in der unter a) hergestellten Zusammensetzung in einem Gesamtgehalt bezogen auf Gewicht von 1% bis 50%, vorzugsweise von 15% bis 40%, stärker bevorzugt von 20% bis 40%, am stärksten bevorzugt von 25% bis 35%, bezogen auf das Gesamtgewicht der unter a) hergestellten Zusammensetzung, vorliegt.

**3.** Zusammensetzung nach einem der vorhergehenden Ansprüche, wobei die unter a) hergestellte Zusammensetzung außerdem mindestens einen Füllstoff umfasst, der aus der Gruppe bestehend aus Füllstoffen pflanzlichen Ursprungs, Füllstoffen mineralischen Ursprungs, Füllstoffen synthetischen Ursprungs und deren Gemischen, vorzugsweise aus der Gruppe bestehend aus Füllstoffen pflanzlichen Ursprungs, Füllstoffen mineralischen Ursprungs und deren Gemischen, stärker bevorzugt aus Füllstoffen pflanzlichen Ursprungs ausgewählt ist.

**4.** Zusammensetzung nach Anspruch 3, wobei:

- die Füllstoffe pflanzlichen Ursprungs aus der Gruppe bestehend aus lignocellulosehaltigen Verbindungen, Holzmehlen, zerkleinerten Fruchtkernen, zerkleinerten Fruchtschalen und deren Gemischen, vorzugsweise aus lignocellulosehaltigen Verbindungen, stärker bevorzugt aus Cellulosen, noch stärker bevorzugt aus Cellulosefasern, am stärksten bevorzugt aus Cellulosefasern, die aus Flachs, Hanf, Hafer, Holz, Schilfrohr, Elefantengras, Bambus oder deren Gemischen stammen, ausgewählt sind und/oder
- die Füllstoffe mineralischen Ursprungs aus der Gruppe bestehend aus Tonen, Glimmern, Talk, Kreiden, Calciumcarbonat, Magnesiumcarbonat, Borsilikaten, Perliten, Kieselerden, Bariumsulfat und deren Gemischen ausgewählt sind.

5. Zusammensetzung nach Anspruch 3 oder 4, wobei der Füllstoff in der unter a) hergestellten Zusammensetzung vorzugsweise in einem Gesamtgehalt bezogen auf Gewicht von 1% bis 50%, vorzugsweise von 1% bis 30%, stärker bevorzugt von 5 % bis 15%, bezogen auf das Gesamtgewicht der unter a) hergestellten Zusammensetzung, vorliegt.

6. Zusammensetzung nach einem der vorhergehenden Ansprüche, wobei der Weichmacher aus der Gruppe bestehend aus Zitronensäureestern, Polyolen, Polyolefinen, Pflanzenölen, Tensiden und deren Gemischen ausgewählt ist.

7. Zusammensetzung nach einem der vorhergehenden Ansprüche, wobei der Weichmacher aus der Gruppe bestehend aus Triethylcitrat, Tributylcitrat, Tributylacetylcitrat, Triethylacetylcitrat und deren Gemischen, vorzugsweise Tributylcitrat, ausgewählt ist.

8. Zusammensetzung nach einem der vorhergehenden Ansprüche, wobei das thermoplastische Polymer aus der Gruppe bestehend aus Poly(3-hydroxybutyrat), Poly(3-hydroxyhexanoat), Poly(3-hydroxyvalerat), Poly(3-hydroxybutyrat-co-3-hydroxyvalerat), Poly(3-hydroxybutyrat-co-3-hydroxyhexanoat), Poly(3-hydroxybutyrat-co-3-hydroxyvalerat) und deren Gemischen, vorzugsweise Poly(3-hydroxybutyrat), ausgewählt ist.

9. Zusammensetzung nach einem der vorhergehenden Ansprüche, wobei das thermoplastische Polymer eine Molmasse von 300 000 g/mol bis 400 000 g/mol aufweist.

10. Zusammensetzung nach einem der vorhergehenden Ansprüche, wobei die unter a) hergestellte Zusammensetzung außerdem mindestens ein Polymer umfasst, ausgewählt aus der Gruppe bestehend aus:

   i) Homopolymeren oder Copolymeren, ausgewählt aus der Gruppe bestehend aus Derivaten von Polymilchsäure, Polycaprolacton, Polyglykolsäure und deren Gemischen;
   ii) aliphatischen und/oder aromatischen Copolyestern;
   iii) Stärke und/oder thermoplastischer Stärke (TPS);
   iv) thermoplastischen Cellulosederivaten (TPC), ausgewählt aus der Gruppe bestehend aus Celluloseestern, Celluloseethern, Alkanoaten und deren Gemischen

   und Gemischen der Verbindungen i) bis iv).

11. Zusammensetzung nach einem der vorhergehenden Ansprüche, wobei die unter a) erhaltene Zusammensetzung außerdem mindestens einen Keimbildner umfasst, ausgewählt aus der Gruppe bestehend aus Bornitrid, Uracil, Thymin, Cytosin, Orotsäure, Ammoniumchlorid, Cyclodextrinen, Polyvinylalkoholpartikeln, Terbiumoxid, Saccharin und deren Gemischen.

12. Zusammensetzung nach einem der vorhergehenden Ansprüche, wobei die unter a) erhaltene Zusammensetzung außerdem mindestens ein Pigment und/oder mindestens einen Farbstoff umfasst, ausgewählt aus der Gruppe bestehend aus Titandioxid, Zinkoxid, Eisenoxiden, Ferrocyanidsalzen, organischen Azopigmenten oder Anthrachinonen und deren Gemischen, vorzugsweise aus der Gruppe bestehend aus Titandioxid, Zinkoxid und deren Gemisch.

13. Zusammensetzung nach einem der vorhergehenden Ansprüche, wobei mindestens ein Teil, vorzugsweise die Gesamtheit der unter a) hergestellten Zusammensetzung in Form von Granulaten hergestellt wird, wobei die Granulate vorzugsweise durch ein Kompaktierungs- oder Compoundierungsverfahren, vorzugsweise durch ein Kompaktierungsverfahren hergestellt werden.

14. Zusammensetzung nach einem der vorhergehenden Ansprüche, wobei Schritt b) des Verfahrens die folgenden Teilschritte umfasst:

   b$_1$) Erhalten eines schmelzextrudierten Gemischs der unter a) hergestellten Zusammensetzung und

b2) Überführen des unter $b_1$) erhaltenen schmelzflüssigen Gemischs zu einer Unterwasserheißabschlagvorrichtung (6), die eine mit einem Unterwasserschneidkopf (8) verbundene Spinndüse (7) umfasst, wobei die Spinndüse (7) eine Mehrzahl von Öffnungen umfasst.

15. Zusammensetzung nach dem vorhergehenden Anspruch, wobei in Schritt $b_2$) das unter $b_1$) erhaltene schmelzflüssige Gemisch den Öffnungen der Spinndüse (7) unter einem Druck zwischen 2000000 Pa und 24000000 Pa, vorzugsweise zwischen 4000000 Pa und 13000000 Pa zugeführt wird.

16. Zusammensetzung nach Anspruch 14 oder 15, wobei sämtliche Öffnungen der Spinndüse (7) dieselbe Form und die gleichen Abmessungen haben, wobei die Form vorzugsweise konisch oder zylindrisch, stärker bevorzugt zylindrisch ist.

17. Zusammensetzung nach einem der Ansprüche 14 bis 16, wobei:

- der maximale Durchmesser der Öffnungen der Spinndüse (7) zwischen 0,25 mm und 1,0 mm beträgt und
- die Länge der Öffnungen der Spinndüse (7) zwischen 2,0 mm und 10,0 mm beträgt.

18. Zusammensetzung nach einem der vorhergehenden Ansprüche, wobei die Partikel:

- eine Rundheit von 0,90 bis 1, vorzugsweise von 0,98 bis 1 und
- ein Formverhältnis von 0,80 bis 1, vorzugsweise von 0,87 bis 1 und
- eine Größe von 250 $\mu$m bis 1000 $\mu$m, vorzugsweise von 250 $\mu$m bis 500 $\mu$m besitzen.

19. Kosmetische Verwendung thermoplastischer Polymerpartikel nach einem der vorhergehenden Ansprüche als Exfoliationsmittel für die menschliche Haut.

20. Verfahren zur kosmetischen Behandlung sichtbarer und/oder tastbarer Unregelmäßigkeiten der menschlichen Haut, umfassend die folgenden Schritte:

i) topische Anwendung einer Kosmetikzusammensetzung nach einem der Ansprüche 1 bis 18 auf die Haut und
ii) Belassen der Zusammensetzung in Kontakt mit der Haut für eine Dauer zwischen 1 min und 30 min, vorzugsweise zwischen 1 min und 5 min und
iii) Beseitigung der Zusammensetzung durch Spülen.

**Claims**

1. Cosmetic composition comprising, in a cosmetically acceptable medium, thermoplastic polymer particles obtained by a process comprising the following steps:

a) preparation of a composition comprising :

- at least one thermoplastic polymer chosen from the group consisting of homopolymers of hydroxyalkanoates, copolymers of hydroxyalkanoates and mixtures thereof; and
- at least one plasticizer; and

b) obtaining thermoplastic polymer particles from the composition prepared in a) by a water-based wet granulation process.

2. Composition according to claim 1 wherein :

- the thermoplastic polymer is present in the composition prepared in a) in a total weight content ranging from 50% to 99%, preferably from 60% to 85%, more preferably from 60% to 80%, most preferably from 65% to 75%, relative to the total weight of the composition prepared in a); and/or
- the plasticizer is present in the composition prepared in a) in a total weight content ranging from 1% to 50%, preferably from 15% to 40%, more preferably from 20% to 40%, most preferably from 25% to 35%, based on the total weight of the composition prepared in a).

3. Composition according to any one of the preceding claims wherein the composition prepared in a) further comprises at least one filler selected from the group consisting of fillers of vegetable origin, fillers of mineral origin, fillers of synthetic origin and mixtures thereof, preferably from the group consisting of fillers of vegetable origin, fillers of mineral origin and mixtures thereof, more preferably from fillers of vegetable origin.

4. Composition according to claim 3 wherein:

   - the fillers of vegetable origin are selected from the group consisting of ligno-cellulosic compounds, wood flours, crushed fruit stones, crushed fruit peels and mixtures thereof, preferably among ligno-cellulosic compounds, more preferably among celluloses, even more preferably among fibrils of cellulose, most preferably among fibrils of celluloses derived from flax, hemp, oats, wood, reed, miscanthus, bamboo or mixtures thereof; and/or
   - the fillers of mineral origin are selected from the group consisting of clays, micas, talc, chalks, calcium carbonate, magnesium carbonate, borosilicates, perlites, silicas, barium sulphate and mixtures thereof.

5. Composition according to claim 3 or 4, wherein the filler is present in the composition prepared in a) preferably in a total weight content ranging from 1% to 50%, preferably from 1% to 30%, more preferably from 5% to 15%, relative to the total weight of the composition prepared in a).

6. A composition according to any one of the preceding claims, wherein the plasticizer is selected from the group consisting of citric acid esters, polyols, polyolefins, vegetable oils, surfactants and mixtures thereof.

7. A composition according to any one of the preceding claims, wherein the plasticizer is selected from the group consisting of triethyl citrate, tributyl citrate, tributyl acetylcitrate, triethyl acetylcitrate and mixtures thereof, preferably tributyl citrate.

8. A composition according to any one of the preceding claims wherein the thermoplastic polymer is selected from the group consisting of poly(3-hydroxybutyrate), poly(3-hydroxyhexanoate), poly(3-hydroxyvalerate), poly(3-hydroxybutyrate-co-3-hydroxyvalerate), poly(3-hydroxybutyrate-co-3-hydroxyhexanoate), poly(3-hydroxybutyrate-co-3-hydroxyvalerate) and mixtures thereof, preferably poly(3-hydroxybutyrate).

9. A composition according to any one of the preceding claims, wherein the thermoplastic polymer has a molar mass ranging from 300,000 g/mol to 400,000 g/mol.

10. A composition according to any one of the preceding claims, wherein the composition prepared in a) further comprises at least one polymer selected from the group consisting of :

    i) homopolymers or copolymers selected from the group consisting of polylactic acid derivatives, polycaprolactone, polyglycolic acid and mixtures thereof;
    ii) aliphatic and/or aromatic co-polyesters;
    iii) starch and/or thermoplastic starch (TPS);
    iv) thermoplastic cellulose derivatives (TPC) selected from the group consisting of cellulose esters, cellulose ethers, alkanoates and mixtures thereof;

    and mixtures of compounds i) to iv).

11. A composition according to any one of the preceding claims wherein the composition obtained in a) further comprises at least one nucleating agent selected from the group consisting of boron nitride, uracil, thymine, cytosine, orotic acid, ammonium chloride, cyclodextrins, polyvinyl alcohol particles, terbium oxide, saccharin and mixtures thereof.

12. A composition according to any one of the preceding claims wherein the composition obtained in a) further comprises at least one pigment and/or at least one colorant selected from the group consisting of titanium dioxide, zinc oxide, iron oxides, ferrocyanide salts, azo or anthraquinone organic pigments and mixtures thereof, preferably from the group consisting of titanium dioxide, zinc oxide and mixtures thereof.

13. A composition according to any one of the preceding claims, wherein at least a portion, preferably the entire composition prepared in a) is prepared in the form of granules, the granules being prepared by a compaction or compounding process, preferably by a compaction process.

**14.** Composition according to any one of the preceding claims, wherein step b) of the process comprises the following sub-steps :

b1) obtaining a molten mixture by extrusion of the composition prepared in a); and
b2) transferring the molten mixture obtained in b1) to an underwater head cutting device (6) comprising a die (7) connected to an underwater cutting head (8), the die (7) comprising a plurality of orifices.

**15.** Composition according to the preceding claim, wherein during step b2), the molten mixture obtained in b1) is fed into the orifices of the die (7) at a pressure of between $2.10^6$ Pa and $24.10^6$ Pa, preferably between $4.10^6$ Pa and $13.10^6$ Pa.

**16.** Composition according to claim 14 or 15, wherein all the orifices of the die (7) have the same shape and the same dimensions, the shape being preferably conical or cylindrical, more preferably cylindrical.

**17.** A composition according to any one of claims 14 to 16, wherein :

- the maximum diameter of the orifices of the die (7) is between 0.25 mm and 1.0 mm; and
- the length of the orifices of the die (7) is between 2,0 mm and 10,0 mm.

**18.** A composition according to any one of the preceding claims, wherein the particles have:

- a circularity ranging from 0.90 to 1, preferably from 0.98 to 1; and
- an aspect ratio ranging from 0.80 to 1, preferably from 0.87 to 1; and
- a size ranging from 250 $\mu$m to 1000 $\mu$m, preferably from 250 $\mu$m to 500 $\mu$m.

**19.** Cosmetic use of thermoplastic polymer particles as defined in any one of the preceding claims as agents for exfoliating human skin.

**20.** Process for the cosmetic treatment of visible and/or tactile irregularities of the human skin comprising the following steps:

i) topical application to the skin of a cosmetic composition as defined in any one of claims 1 to 18; and
(ii) maintaining the composition in contact with the skin for a period of between 1 min and 30 min, preferably between 1 min and 5 min; and
(iii) removing the composition by rinsing.

FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1247574

- US 20170218119 A